(19)

Europäisches
Patentamt

European
Patent Office

Office européen
des brevets

(11)     **EP 1 636 226 B1**

(12)                        **EUROPEAN PATENT SPECIFICATION**

(45) Date of publication and mention
of the grant of the patent:
**15.10.2014 Bulletin 2014/42**

(21) Application number: **04731161.8**

(22) Date of filing: **05.05.2004**

(51) Int Cl.:
*C07D 471/04* (2006.01)        *A61K 31/437* (2006.01)
*A61P 7/02* (2006.01)

(86) International application number:
**PCT/EP2004/004754**

(87) International publication number:
**WO 2004/101563 (25.11.2004 Gazette 2004/48)**

(54) **Azaindole derivatives as Factor Xa inhibitors**

Azaindol-Derivate als Faktor Xa Hemmer

Dérivés azaindole en tant qu'inhibiteurs du Facteur Xa

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(30) Priority: **19.05.2003 EP 03011304**

(43) Date of publication of application:
**22.03.2006 Bulletin 2006/12**

(73) Proprietor: **Sanofi-Aventis Deutschland GmbH
65929 Frankfurt am Main (DE)**

(72) Inventors:
• **NAZARE, Marc
65510 Idstein (DE)**
• **WEHNER, Volkmar
97657 Sandberg (DE)**

• **WILL, David, William
65926 Frankfurt am Main (DE)**
• **RITTER, Kurt
60594 Frankfurt Am Main (DE)**
• **URMANN, Matthias
65760 Eschborn (DE)**
• **MATTER, Hans
63505 Langenselbold (DE)**

(56) References cited:
**WO-A-01/64639          WO-A-99/33800
WO-A-02/070523        WO-A-03/044014
WO-A-03/053361        WO-A1-99/32477
WO-A1-03/084533      WO-A2-01/19788
WO-A2-02/00651        WO-A2-02/080853
WO-A2-2004/083177  JP-A- 2004 203 791
JP-A- 2004 210 716    US-A1- 2002 091 116**

**Description**

The present invention relates to compounds defined in claim 1. These compounds are valuable pharmacologically active compounds. They exhibit a strong antithrombotic effect and are suitable, for example, for the therapy and prophylaxis of cardiovascular disorders like thromboembolic diseases or restenoses. They are reversible inhibitors of the blood clotting enzymes factor Xa (FXa) and/or factor VIIa (FVIIa), and can in general be applied in conditions in which an undesired activity of factor Xa and/or factor VIIa is present or for the cure or prevention of which an inhibition of factor Xa and/or factor VIIa is intended. The invention furthermore relates to processes for the preparation of compounds defined in claim 1, their use, in particular as active ingredients in pharmaceuticals, and pharmaceutical preparations comprising them.

Normal haemeostasis is the result of a complex balance between the processes of clot initiation, formation and clot dissolution. The complex interactions between blood cells, specific plasma proteins and the vascular surface, maintain the fluidity of blood unless injury and blood loss occurs (EP-A-987274). Many significant disease states are related to abnormal haemeostasis. For example, local thrombus formation due to rupture of atheroslerotic plaque is a major cause of acute myocardial infarction and unstable angina. Treatment of an occlusive coronary thrombus by either thrombolytic therapy or percutaneous angioplasty may be accompanied by acute thrombolytic reclosure of the affected vessel.

There continues to be a need for safe and effective therapeutic anticoagulants to limit or prevent thrombus formation. It is most desirable to develop agents that inhibit coagulation without directly inhibiting thrombin but by inhibiting other steps in the coagulation cascade like factor Xa and/or factor VIIa activity. It is now believed that inhibitors of factor Xa carry a lower bleeding risk than thrombin inhibitors (A. E. P. Adang & J. B. M. Rewinkel, Drugs of the Future 2000, 25, 369-383).

Low molecular weight, factor Xa-specific blood clotting inhibitors that are effective but do not cause unwanted side effects have been described, for example, in WO-A-95/29189.

However, besides being an effective factor Xa-specific blood clotting inhibitor, it is desirable that such inhibitors also have further advantageous properties, for instance stability in plasma and liver and selectivity versus other serine proteases whose inhibition is not intended, such as thrombin. There is an ongoing need for further low molecular weight factor Xa specific blood clotting inhibitors, which are effective and have the above advantages as well.

Specific inhibition of the factor VIIa/tissue factor catalytic complex using monoclonal antibodies (WO-A-92/06711) or a protein such as chloromethyl ketone inactivated factor VIIa (WO-A-96/12800, WO-A-97/47651) is an extremely effective means of controlling thrombus formation caused by acute arterial injury or the thrombotic complications related to bacterial septicemia. There is also experimental evidence suggesting that inhibition of factor VIIa/tissue factor activity inhibits restenosis following balloon angioplasty. Bleeding studies have been conducted in baboons and indicate that inhibition of the factor VIIa/tissue factor complex has the widest safety window with respect to therapeutic effectiveness and bleeding risk of any anticoagulant approach tested including thrombin, platelet and factor Xa inhibition. Certain inhibitors of factor VIIa have already been described. EP-A-987274, for example discloses compounds containing a tripeptide unit, which inhibit factor VIIa. However, the property profile of these compounds is still not ideal, and there is an ongoing need for further low molecular weight factor VIIa inhibitory blood clotting inhibitors

The present invention satisfies the above needs by providing novel compounds, which exhibit better factor Xa and/or factor VIIa inhibitory activity and are favorable agents with high bioavailability.

Thus the present invention relates to compounds in all its stereoisomeric forms and mixtures thereof in any ratio, and its physiologically tolerable salts, wherein the compound is

1-[5-(5-Chloro-thiophen-2-yl)-isoxazol-3-ylmethyl]-1H-pyrrolo[2,3-b]pyridine-2-carboxylic acid (1-isopropyl-piperidin-4-yl)-amide,

1-[5-(5-Chloro-thiophen-2-yl)-isoxazol-3-ylmethyl]-2-(1-isopropyl-piperidin-4-ylcarbamoyl)-1 H-pyrrolo[2,3-b]pyridine-5-carboxylic acid methyl ester,

1-[5-(5-Chloro-thiophen-2-yl)-isoxazol-3-ylmethyl]-2-(1-isopropyl-piperidin-4-ylcarbamoyl)-1H-pyrrolo[2,3-b]pyridine-5-carboxylic acid,

1-[5-(5-Chloro-thiophen-2-yl)-isoxazol-3-ylmethyl]-1H-pyrrolo[2,3-b]pyridine-2,5- dicarboxylic acid 5-amide 2-[(1-isopropyl-piperidin-4-yl)-amide],

1-[5-(5-Chloro-thiophen-2-yl)-isoxazol-3-ylmethyl]-1H-pyrrolo[3,2-b]pyridine-2-carboxylic acid (1-isopropyl-piperidin-4-yl)-amide, or

1-[5-(5-Chloro-thiophen-2-yl)-isoxazol-3-ylmethyl]-2-(1-isopropyl-piperidin-4-ylcarbamoyl)-1H-pyrrolo[3,2-b]pyridine-5-carboxylic acid ,

Optically active carbon atoms present in the compounds defined in claim 1 can independently of each other have R configuration or S configuration. The compounds defined in claim 1 can be present in the form of pure enantiomers or pure diastereomers or in the form of mixtures of enantiomers and/or diastereomers, for example in the form of racemates. The present invention relates to pure enantiomers and mixtures of enantiomers as well as to pure diastereomers and mixtures of diastereomers. The invention comprises mixtures of two or of more than two stereoisomers of these compounds, and it comprises all ratios of the stereoisomers in the mixtures. In case the compounds can be present as E isomers or Z isomers (or cis isomers or trans isomers) the invention relates both to pure E isomers and pure Z isomers and to E/Z mixtures in all ratios. The invention also comprises all tautomeric forms of the compounds defined in claim 1.

Diastereomers, including E/Z isomers, can be separated into the individual isomers, for example, by chromatography. Racemates can be separated into the two enantiomers by customary methods, for example by chromatography on chiral phases or by resolution, for example by crystallization of diastereomeric salts obtained with optically active acids or bases. Stereochemically uniform compounds defined in claim 1 can also be obtained by employing stereochemically uniform starting materials or by using stereoselective reactions.

Physiologically tolerable salts of the compounds defined in claim 1 are nontoxic salts that are physiologically acceptable, in particular pharmaceutically utilizable salts. Such salts of these compounds containing acidic groups, for example a carboxyl group COOH, are for example alkali metal salts or alkaline earth metal salts such as sodium salts, potassium salts, magnesium salts and calcium salts, and also salts with physiologically tolerable quaternary ammonium ions such as tetramethylammonium or tetraethylammonium, and acid addition salts with ammonia and physiologically tolerable organic amines, such as methylamine, dimethylamine, trimethylamine, ethylamine, triethylamine, ethanolamine or tris-(2-hydroxyethyl)amine. Basic groups contained in the compounds defined in claim 1, for example amino groups or guanidino groups, form acid addition salts, for example with inorganic acids such as hydrochloric acid, hydrobromic acid, sulfuric acid, nitric acid or phosphoric acid, or with organic carboxylic acids and sulfonic acids such as formic acid, acetic acid, oxalic acid, citric acid, lactic acid, malic acid, succinic acid, malonic acid, benzoic acid, maleic acid, fumaric acid, tartaric acid, methanesulfonic acid or p-toluenesulfonic acid. Compounds defined in claim 1, which simultaneously contain a basic group and an acidic group, for example a guanidino group and a carboxyl group, can also be present as zwitterions (betaines) which are likewise included in the present invention.

Salts of compounds defined in claim 1 can be obtained by customary methods known to those skilled in the art, for example by combining a compound defined in claim 1 with an inorganic or organic acid or base in a solvent or dispersant, or from other salts by cation exchange or anion exchange. The present invention also includes all salts of the compounds defined in claim 1 which, because of low physiologically tolerability, are not directly suitable for use in pharmaceuticals but are suitable, for example, as intermediates for carrying out further chemical modifications of the compounds or as starting materials for the preparation of physiologically tolerable salts.

The present invention furthermore includes all solvates of compounds defined in claim 1, for example hydrates or adducts with alcohols.

The compounds defined in claim 1 can be prepared by utilising procedures and techniques, which per se are well known and appreciated by one of ordinary skill in the art. Starting materials or building blocks for use in the general synthetic procedures that can be applied in the preparation of the compounds defined in claim 1 are readily available to one of ordinary skill in the art. In many cases they are commercially available or have been described in the literature. Otherwise they can be prepared from readily available precursor compounds analogously to procedures described in the literature, or by procedures or analogously to procedures described in this application.

In general, compounds defined in claim 1 can be prepared, for example in the course of a convergent synthesis, by linking two or more fragments which can be derived retrosynthetically from the compound. More specifically, suitably substituted starting azaindole derivatives are employed as building blocks in the preparation of the compounds defined in claim 1. Although various synthetic aspects of the azaindole chemistry are considerably different to the indole chemistry many procedures describing the synthesis and functionalisation of indoles can be modified and adopted by those skilled in the art. Therefore literature describing transformations and the synthesis of indoles are highly instructive and applicable to the azaindole chemistry. If not commercially available, such azaindole derivatives can be prepared according to the well-known standard procedures for the formation of the azaindole ring system such as, for example, the Fischer indole synthesis, the Bischler indole synthesis, or the Reissert indole synthesis. By choosing suitable precursor molecules, these azaindole syntheses allow the introduction of a variety of substituents into the various positions of the azaindole system, which can then be chemically modified in order to finally arrive at the molecule defined in claim 1 having the desired substituent pattern. As one of the comprehensive reviews in which numerous details and literature references on the chemistry of indoles and on synthetic procedures for their preparation can be found, W. J. Houlihan (ed.), "Indoles, Part One", volume 25, 1972, out of the series "The Chemistry of Heterocyclic Compounds", A. Weissberger and E. C. Taylor (ed.), John Wiley & Sons; R.E. Willette, Advances in Heterocyclic Chemistry 9 (1968) 27; J.-Y. Mérour Curr. Org.

Chem. 5 (2001) 471; H. Döpp et al. in Houben-Weyl, "Methoden der Organischen Chemie" (Methods of Organic Chemistry), Georg Thieme Verlag, Stuttgart, Germany 1994, Vol E6a,b part 2a Hetarene I, is referred to.

If starting azaindole derivatives are to be synthesized this can be done, for example, according to the well-known azaindole syntheses mentioned above. In the following they are explained briefly, however, they are standard procedures comprehensively discussed in the literature, and are well known to one skilled in the art.

Preferred methods include, but are not limited to those described in the examples. The compounds of the present invention are serine protease inhibitors, which inhibit the activity of the blood coagulation enzyme factors Xa and/or factor VIIa. In particular, they are highly active inhibitors of factor Xa. They are specific serine protease inhibitors inasmuch as they do not substantially inhibit the activity of other proteases whose inhibition is not desired. The activity of the compounds of the present invention can be determined, for example, in the assays described below or in other assays known to those skilled in the art. With respect to factor Xa inhibition, a preferred embodiment of the invention comprises compounds which have a $K_i < 1$ mM for factor Xa inhibition as determined in the assay described below, with or without concomitant factor VIIa inhibition, and which preferably do not substantially inhibit the activity of other proteases involved in coagulation and fibrinolysis whose inhibition is not desired (using the same concentration of the inhibitor). The compounds of the invention inhibit factor Xa catalytic activity either directly, within the prothrombinase complex or as a soluble subunit, or indirectly, by inhibiting the assembly of factor Xa into the prothrombinase complex.

As inhibitors of factor Xa and/or factor VIIa the compounds of the invention and their physiologically tolerable salts are generally suitable for the therapy and prophylaxis of conditions in which the activity of factor Xa and/or factor VIIa plays a role or has an undesired extent, or which can favorably be influenced by inhibiting factor Xa and/or factor VIIa or decreasing their activities, or for the prevention, alleviation or cure of which an inhibition of factor Xa and/or factor VIIa or a decrease in their activity is desired by the physician. As inhibition of factor Xa and/or factor VIIa influences blood coagulation and fibrinolysis, the compounds of the invention and their physiologically tolerable salts are generally suitable for reducing blood clotting, or for the therapy and prophylaxis of conditions in which the activity of the blood coagulation system plays a role or has an undesired extent, or which can favorably be influenced by reducing blood clotting, or for the prevention, alleviation or cure of which a decreased activity of the blood coagulation system is desired by the physician. A specific subject of the present invention thus are the reduction or inhibition of unwanted blood clotting, in particular in an individual, by administering an effective amount of a compound or a physiologically tolerable salt, as well as pharmaceutical preparations therefor.

The invention also relates to the treatment of disease states such as abnormal thrombus formation, acute myocardial infarction, unstable angina, thromboembolism, acute vessel closure associated with thrombolytic therapy or percutaneous transluminal coronary angioplasty (PTCA), transient ischemic attacks, stroke, intermittent claudication or bypass grafting of the coronary or peripheral arteries, vessel luminal narrowing, restenosis post coronary or venous angioplasty, maintenance of vascular access patency in long-term hemodialysis patients, pathologic thrombus formation occurring in the veins of the lower extremities following abdominal, knee or hip surgery, pathologic thrombus formation occurring in the veins of the lower extremities following abdominal, knee and hip surgery, a risk of pulmonary thromboembolism, or disseminated systemic intravascular coagulatopathy occurring in vascular systems during septic shock, certain viral infections or cancer.

The compounds of the present invention can also be used to reduce an inflammatory response. Examples of specific disorders for the treatment or prophylaxis of which the compounds of the invention can be used are coronary heart disease, myocardial infarction, angina pectoris, vascular restenosis, for example restenosis following angioplasty like PTCA, adult respiratory distress syndrome, multi-organ failure and disseminated intravascular clotting disorder. Examples of related complications associated with surgery are thromboses like deep vein and proximal vein thrombosis, which can occur following surgery.

The compounds of the invention and their physiologically tolerable salts can be administered to animals, preferably to mammals, and in particular to humans as pharmaceuticals for therapy or prophylaxis. They can be administered on their own, or in mixtures with one another or in the form of pharmaceutical preparations, which permit enteral or parenteral administration.

The pharmaceuticals can be administered orally, for example in the form of pills, tablets, lacquered tablets, coated tablets, granules, hard and soft gelatin capsules, solutions, syrups, emulsions, suspensions or aerosol mixtures. Administration, however, can also be carried out rectally, for example in the form of suppositories, or parenterally, for example intravenously, intramuscularly or subcutaneously, in the form of injection solutions or infusion solutions, microcapsules, implants or rods, or percutaneously or topically, for example in the form of ointments, solutions or tinctures, or in other ways, for example in the form of aerosols or nasal sprays.

The pharmaceutical preparations according to the invention are prepared in a manner known per se and familiar to one skilled in the art, pharmaceutically acceptable inert inorganic and/or organic carriers being used in addition to the compound(s) of the invention and/or its (their) physiologically tolerable salts. For the production of pills, tablets, coated tablets and hard gelatin capsules it is possible to use, for example, lactose, cornstarch or derivatives thereof, talc, stearic acid or its salts, etc. Carriers for soft gelatin capsules and suppositories are, for example, fats, waxes, semisolid and liquid polyols, natural or hardened oils, etc. Suitable carriers for the production of solutions, for example injection solutions, or of emulsions or syrups are, for example, water, saline, alcohols, glycerol, polyols, sucrose, invert sugar, glucose, vegetable oils, etc. Suitable carriers for microcapsules, implants or rods are, for example, copolymers of glycolic acid and lactic acid. The pharmaceutical preparations normally contain about 0.5 % to 90 % by weight of the compounds of the finvention and/or their physiologically tolerable salts. The amount of the active ingredient and/or its physiologically tolerable salts in the pharmaceutical preparations normally is from about 0.5 mg to about 1000 mg, preferably from about 1 mg to about 500 mg.

In addition to the active ingredients and/or their physiologically acceptable salts and to carrier substances, the pharmaceutical preparations can contain additives such as, for example, fillers, disintegrants, binders, lubricants, wetting agents, stabilizers, emulsifiers, preservatives, sweeteners, colorants, flavorings, aromatizers, thickeners, diluents, buffer substances, solvents, solubilizers, agents for achieving a depot effect, salts for altering the osmotic pressure, coating agents or antioxidants. They can also contain two or more compounds of the invention, and/or their physiologically tolerable salts. In case a pharmaceutical preparation contains two or more compounds of the invention, the selection of the individual compounds can aim at a specific overall pharmacological profile of the pharmaceutical preparation. For example, a highly potent compound with a shorter duration of action may be combined with a long-acting compound of lower potency.

When using the compounds of the invention the dose can vary within wide limits and, as is customary and is known to the physician, is to be suited to the individual conditions in each individual case. It depends, for example, on the specific compound employed, on the nature and severity of the disease to be treated, on the mode and the schedule of administration, or on whether an acute or chronic condition is treated or whether prophylaxis is carried out. An appropriate dosage can be established using clinical approaches well known in the medical art. In general, the daily dose for achieving the desired results in an adult weighing about 75 kg is from 0.01 mg/kg to 100 mg/kg, preferably from 0.1 mg/kg to 50 mg/kg, in particular from 0.1 mg/kg to 10 mg/kg, (in each case in mg per kg of body weight). The daily dose can be divided, in particular in the case of the administration of relatively large amounts, into several, for example 2, 3 or 4, part administrations. As usual, depending on individual behavior it may be necessary to deviate upwards or downwards from the daily dose indicated.

A compound of the invention can also advantageously be used as an anticoagulant outside an individual. For example, an effective amount of a compound of the invention can be contacted with a freshly drawn blood sample to prevent coagulation of the blood sample. Further, a compound of the invention or its salts can be used for diagnostic purposes, for example in in vitro diagnoses, and as an auxiliary in biochemical investigations. For example, a compound of the invention can be used in an assay to identify the presence of factor Xa and/or factor VIIa or to isolate factor Xa and/or factor VIIa in a substantially purified form. A compound of the invention can be labeled with, for example, a radioisotope, and the labeled compound bound to factor Xa and/or factor VIIa is then detected using a routine method useful for detecting the particular label. Thus, a compound of the invention or a salt thereof can be used as a probe to detect the location or amount of factor Xa and/or factor VIIa activity in vivo, in vitro or ex vivo.

Furthermore, the compounds of the invention can be used as synthesis intermediates for the preparation of other compounds, in particular of other pharmaceutical active ingredients, which are obtainable from the compounds of the invention, for example by introduction of substituents or modification of functional groups.

The general synthetic sequences for preparing the compounds useful in the present invention our outlined in the examples given below. Both an explanation of, and the actual procedure for, the various aspects of the present invention are described where appropriate. The following examples are intended to be merely illustrative of the present invention, and not limiting thereof in either scope or spirit. Those with skill in the art will readily understand that known variations of the conditions and processes described in the examples can be used to synthesize the compounds of the present invention.

It is understood that changes that do not substantially affect the activity of the various embodiments of this invention are included within the invention disclosed herein. Thus, the following examples are intended to illustrate but not limit the present invention.

Examples

When in the final step of the synthesis of a compound an acid such as trifluoroacetic acid or acetic acid was used, for example when trifluoroacetic acid was employed to remove a tBu group or when a compound was purified by chromatography using an eluent which contained such an acid, in some cases, depending on the work-up procedure, for example the details of a freeze-drying process, the compound was obtained partially or completely in the form of a salt of the acid used, for example in the form of the acetic acid salt or trifluoroacetic acid salt or hydrochloric acid salt.

Abbreviations used:

| tert-Butyl | tBu |
|---|---|
| 2,2'-bis(diphenylphoshino-1,1'-binaphthyl | Binap |
| Bis-(oxo-3-oxazolidinyl)-phosphoryl chloride | BOP-Cl |
| dibenzylidenacetone | dba |
| Dichloromethane | DCM |
| Dicyclohexyl-carbodiimide | DCC |
| Diethylphosphoryl cyanide | DEPC |
| 4-Dimethyaminopyridine | DMAP |
| N,N-Dimethylformamide | DMF |
| Dimethylsulfoxide | DMSO |
| 1,1'-Bis(diphenylphosphino)ferrocene | DPPF |
| 0-(7-Azabenzotriazol-1-yl)-N,N,N',N'-tetramethyluronium-hexafluorophosphate | HATU |
| N-Bromosuccinimide | NBS |
| N-Chlorosuccinimide | NCS |
| N-Iodosuccinimide | NIS |
| N-Ethylmorpholine | NEM |
| Methanol | MeOH |
| Room temperature 20 °C to 25 °C | RT |
| Saturated | sat. |
| Tetrahydrofuran | THF |
| Trifluoroacetic acid | TFA |
| 0-((Ethoxycarbonyl)cyanomethyleneamino)-N,N,N',N'-tetramethyluronium tetrafluoroborate | TOTU |

Example 1: 1-[5-(5-Chloro-thiophen-2-yl)-isoxazol-3-ylmethyl]-1H-pyrrolo[2,3-b]pyridine-2-carboxylic acid (1-isopropyl-piperidin-4-yl)-amide

(i) 1H-Pyrrolo[2,3-b]pyridine-2-carboxylic acid methyl ester

0.495 g (1.64 mmol) of 1-benzenesulfonyl-1 H-pyrrolo[2,3-b]pyridine-2-carboxylic acid was dissolved in 5 ml of methanol and 3 ml of 2N aqueous sodium hydroxide. The reaction was stirred at 40°C for 8 h. The solvent was removed under reduced pressure. Residual volatiles were removed by twice codistilling with toluene. The residue was suspended in methanolic hydrochloric acid and stirred for 16h at RT. The solvent was removed under reduced pressure. The residue was dissolved in ethyl acetate and washed with saturated aqueous sodium bicarbonate solution, and saturated aqueous sodium chloride solution. The organic phase was dried with sodium sulfate, filtered and the solvent was removed under reduced pressure. Yield 0.201 g. MS (Cl+): m/e = 177 (M+H$^+$).

(ii) 1-[5-(5-Chloro-thiophen-2-yl)-isoxazol-3-ylmethyl]-1H-pyrrolo[2,3-b]pyridine-2- carboxylic acid

0.195 g (1.1 mmol) of 1H-pyrrolo[2,3-b]pyridine-2-carboxylic acid methyl ester was dissolved in 4 ml of DMF and 48.7mg (1.2 mmol) of sodium hydride (60% in mineral oil) was added. The reaction was stirred at RT for 20 min, cooled to -78°C then 324 mg (1.2 mmol) of 3-bromomethyl-5-(5-chloro-thiophen-2-yl)-isoxazole [prepared by adopting a procedure described by Ewing, William R.; Becker, Michael R.; Choi-Sledeski, Yong Mi; Pauls, Heinz W.; He, Wei; Condon, Stephen M.; Davis, Roderick S.; Hanney, Barbara A.; Spada, Alfred P.; Burns, Christopher J.; Jiang, John Z.; Li, Aiwen; Myers, Michael R.; Lau, Wan F.; Poli, Gregory B; PCT Int. Appl. (2001) 460 pp. WO 0107436 A2] was added. The reaction was allowed to warm to RT overnight. 0.3 ml of 2N aqueous sodium hydroxide was added and the reaction was stirred at RT for 24 h. The product was purified by preparative RP-HPLC eluting with a gradient of 0-100% acetonitrile in water (+0.01% trifluoroacetic acid). After lyophilization the product was obtained as a solid. Yield 280 mg. MS (TOF MS ES+): m/e = 359 (M$^+$).

(iii) (1-Isopropyl-piperidin-4-yl)-carbamic acid tert-butyl ester

To a solution of 5.0 g Piperidin-4-yl-carbamic acid tert-butyl ester in 15 ml methanol, 7.34 ml acetone, 3.14 g

Na(CN)BH$_3$ and 0.3 ml acetic acid were added. After stirring for 16 h at RT the solvent was removed under reduced pressure and the residue was partitioned between 30 ml of water and 30 ml of ethyl acetate. The organic layer was washed with saturated Na$_2$CO$_3$ solution, water and then dried over Na$_2$SO$_4$. Following filtration, the solvent was removed under reduced pressure to yields a white solid. Yield: 4.8g MS (ES$^+$): m/e= 243.

(iv) 1-isopropyl-piperidin-4-ylamine

To 4.8 g (1-Isopropyl-piperidin-4-yl)-carbamic acid tert-butyl ester in 15 ml methanol, 20 ml methanolic hydrochloric acid (8M) were added and the mixture was stirred for 16 h. Removal of the solvent under reduced pressure yielded a white solid, which was coevaporated twice with 20 ml toluene. The product was obtained as its hydrochloride. Yield: 5.42 gMS (ES$^+$): m/e= 143.

(v) 1-[5-(5-Chloro-thiophen-2-yl)-isoxazol-3-ylmethyl]-1H-pyrrolo[2,3-b]pyridine-2- carboxylic acid (1-isopropyl-piperidin-4-yl)-amide

0.135 g (0.4 mmol) of 1-[5-(5-Chloro-thiophen-2-yl)-isoxazol-3-ylmethyl]-1H-pyrrolo[2,3-b]pyridine-2- carboxylic acid, 0.432 g (3.8 mmol) of NEM and 135 mg (0.4 mmol) of TOTU were dissolved in 3 ml of DMF and stirred at RT for 20 min. 89 mg (0.4 mmol) of 1-Isopropyl-piperidin-4-ylamine dihydrochloride salt was added to the reaction solution and stirred at RT for 4 h. The product was purified by preparative RP-HPLC eluting with a gradient of 0-100% acetonitrile in water (+0.01% trifluoroacetic acid). After lyophilization the product was obtained as a solid.
Yield: 156 mg MS (TOF MS ES +): m/e =484 (M$^+$).

Example 2: 1-[5-(5-Chloro-thiophen-2-yl)-isoxazol-3-ylmethyl]-2-(1-isopropyl-piperidin-4-ylcarbamoyl)-1H-pyrrolo[2,3-b]pyridine-5-carboxylic acid methyl ester

(i) 6-Amino-nicotinic acid methyl ester

To a solution of 10 g 6-Amino-nicotinic acid in 100 ml MeOH, 0.8 ml concentrated H$_2$SO$_4$ were added and the mixture was heated to 60 °C for 12 h. Then the reaction mixture was concentrated under reduced pressure. After addition of 50 ml ice water the mixture was brought to pH 8 by addition of K$_2$CO$_3$. The aqueous phase was extracted with ethyl acetate (3x100 ml) and the combined organic layers were dried over MgSO$_4$. Removal of the solvent yielded 5.5 g of the desired product which was subjected to the following reaction without further purification.
Yield: 5.5 g.

(ii) 6-Amino-5-iodo-nicotinic acid methyl ester

To 5 g 6-Amino-nicotinic acid methyl ester and 16.2 g Bis(pyridine)iodonium(I) tetrafluoroborate in 250 ml DCM, 7.6 ml Trifluoromethanesulfonic acid were added dropwise at 0°C. The mixture was stirred for 24 h at RT. Then additional 3.2 g Bis(pyridine)iodonium(I) tetrafluoroborate and 1.5 ml Trifluoromethanesulfonic acid were added. After stirring for 2 h at RT the reaction mixture was concentrated under reduced pressure and then taken-up with concentrated aqueous Na$_2$SO$_3$ solution and brought to pH 8 with concentrated aqueous ammonia. The mixture was extracted with ethyl acetate (2x150 ml). The combined organic layers were washed with brine and then dried over MgSO$_4$. After filtration the solvent was removed under reduced pressure and the residue was codestilled with 100 ml toluene. Yield: 9.6 g.

(iii) 1 H-Pyrrolo[2,3-b]pyridine-2,5-dicarboxylic acid 5-methyl ester

A solution of 5.6 g 6-Amino-5-iodo-nicotinic acid methyl ester, 5.3 g 2-Oxo-propionic acid, 11.1 g NEt$_3$, 4.2 g Triphenylphosphine and 1.1 g Pd(OAc)$_2$ in 100 ml DMF was heated under argon to 100°C. After 10 h the reaction mixture was concentrated under reduced pressure and the residue was stirred with 250 ml water for 1 h. The precipitated product was collected by filtration and washed with water. The crude product was subjected to the next reaction step without further purification. Yield: 10 g.

(iv) 2-(1-Isopropyl-piperidin-4-ylcarbamoyl)-1H-pyrrolo[2,3-b]pyridine-5-carboxylic acid methyl ester

To a solution of 9.5 g 1H-Pyrrolo[2,3-b]pyridine-2,5-dicarboxylic acid 5-methyl ester in 120 ml DMF and 23.9 ml NEt$_3$, 9.2 g 1-Isopropyl-piperidin-4-ylamine hydrochloride and 11 g BOP-Cl were added at RT and the mixture was stirred for 3 h. After addition of 20 ml of water the reaction mixture was extracted with ethyl acetate (3x150 ml). The combined organic layers were washed with brine (1x50 ml) and then dried over MgSO$_4$. After filtration the solvent was

7

removed under reduced pressure and the residue was purified by chromatography on silica gel eluting with EtOAc/MeOH 9:7 -> EtOAc/MeOH/NH3(aq.) 6:4:0.04. The fractions containing the product were evaporated and codestilled with toluene. Yield: 7.2 g.

(v) 1-[5-(5-Chloro-thiophen-2-yl)-isoxazol-3-ylmethyl]-2-(1-isopropyl-piperidin-4-ylcarbamoyl)-1 H-pyrrolo[2,3-b]pyridine-5-carboxylic acid methyl ester

To a solution of 1.2 g 2-(1-Isopropyl-piperidin-4-ylcarbamoyl)-1H-pyrrolo[2;3-b]pyridine-5-carboxylic acid methyl ester in 20 ml DMF, 91 mg sodium hydride (95%) were added at 0°C. Then the reaction mixture was warmed to RT and stirred for 30 min. After cooling again to 0°C, 967 mg 3-Bromomethyl-5-(5-chloro-thiophen-2-yl)-isoxazole [prepared by adopting a procedure described by Ewing, William R.; Becker, Michael R.; Choi-Sledeski, Yong Mi; Pauls, Heinz W.; He, Wei; Condon, Stephen M.; Davis, Roderick S.; Hanney, Barbara A.; Spada, Alfred P.; Burns, Christopher J.; Jiang, John Z.; Li, Aiwen; Myers, Michael R.; Lau, Wan F.; Poli, Gregory B; PCT Int. Appl. (2001), 460 pp. WO 0107436 A2] were added and the mixture was stirred for 2 h at RT. Then 50 ml of water were added and the precipitate was collected by filtration to yield 630 mg pure product. The filtrate was concentrated under reduced pressure and the residue purified by preparative RP-HPLC eluting with a gradient of 0-100% acetonitrile in water (+0.01% trifluoroacetic acid). After lyophilization further 371 mg of product were obtained as a solid.
Yield: 1.0 g MS (ES$^+$): m/e = 542, chloro pattern.

Example 3: 1-[5-(5-Chloro-thiophen-2-yl)-isoxazol-3-ylmethyl]-2-(1-isopropyl-piperidin-4-ylcarbamoyl)-1H-pyrrolo[2,3-b]pyridine-5-carboxylic acid

To a solution of 630 mg of 1-[5-(5-Chloro-thiophen-2-yl)-isoxazol-3-ylmethyl]-2-(1-isopropyl-piperidin-4-ylcarbamoyl)-1H-pyrrolo[2,3-b]pyridine-5-carboxylic acid methyl ester in 60 ml MeOH, 8.7 ml of a 1 M aqueous NaOH solution were added. The reaction mixture was heated to 60°C for 3 h. After cooling to RT 8.8 ml of a 1 M aqueous HCl were added and the solvents were removed under reduced pressure. The residue was stirred with water/MeCN 2:1 and the precipitated product was collected by filtration.
Yield: 276 mg MS (ES$^+$): m/e= 528, chloro pattern.

Example 4: 1-[5-(5-Chloro-thiophen-2-yl)-isoxazol-3-ylmethyl]-1H-pyrrolo[2,3-b]pyridine-2,5-dicarboxylic acid 5-amide 2-[(1-isopropyl-piperidin-4-yl)-amide]

To a solution of 100 mg 1-[5-(5-Chloro-thiophen-2-yl)-isoxazol-3-ylmethyl]-2-(1-isopropyl-piperidin-4-ylcarbamoyl)-1H-pyrrolo[2,3-b]pyridine-5-carboxylic acid in 1 ml DMF, 62 mg TOTU, 0.2 ml DIPEA and 10 mg NH$_4$Cl were added at RT and stirred for 16 h. Then the sovent was removed under reduced pressure and the crude material purified by preparative HPLC (C18 reverse phase column, elution with a H$_2$O/MeCN gradient with 0.1% TFA). The fractions containing the product were evaporated and lyophilized to yield a white solid. The product was obtained as its trifluoroacetate salt. Yield: 16 mg MS (ES$^+$): m/e= 527, chloro pattern.

Example 5: 1-[5-(5-Chloro-thiophen-2-yl)-isoxazol-3-ylmethyl]-1H-pyrrolo[3,2-b]pyridine-2-carboxylic acid (1-isopropyl-piperidin-4-yl)-amide

(i) 1 H-Pyrrolo[3,2-b]pyridine-2-carboxylic acid

1.22 ml 2-oxo-propionic acid, 0.26 g palladium acetate and 3.20 ml triethylamine were added to a solution of 1.00 g 2-bromo-pyridin-3-yl amine and 1.21 g triphenyl-phosphine in 10 ml N,N-dimethylformamide. The reaction mixture was stirred for 4 hours at 100°C. After removal of the solvent under reduced pressure, the residue was purified by column chromatography on silica gel with dichloromethane/ methanol as eluent. Yield: 260 mg MS(ES$^+$): m/e=163. 1H-NMR (400 MHz, DMSO/TMS): δ = 13.30 (s, 1 H); 12.00 (s, 1 H); 8.45 (d, 1 H); 7.82 (d, 1 H); 7.25 (dd, 1 H); 7.14 (s, 1H).

(ii) 1H-Pyrrolo[3,2-b]pyridine-2-carboxylic acid methyl ester

A solution of 130 mg 1H-pyrrolo[3,2-b]pyridine-2-carboxylic acid in 5 ml of a 8 N solution of hydrochloric acid in methanol was stirred at 60°C for 6 hours. Removal of the solvent under reduced pressure yielded a white solid, which was coevaporated twice with 5 ml toluene. The product was obtained as its hydrochloride. Yield: 150 mg MS(ES$^+$): m/e=177.
1H-NMR (400 MHz, DMSO/TMS): δ = 13.60 (s, 1H); 8.86 (d, 1H); 8.59 (d, 1H); 7.82 (dd, 1H); 7.41 (s, 1H); 3.99. (s, 3 H).

(iii) 1-[5-(5-Chloro-thiophen-2-yl)-isoxazol-3-ylmethyl]-1H-pyrrolo[3,2-b]pyridine-2- carboxylic acid methyl ester

To a solution of 150 mg 1H-pyrrolo[3,2-b]pyridine-2-carboxylic acid methyl ester in 2 ml N,N-dimethylformamide 20.4 mg sodium hydride (95%) were added at 0°C. After stirring at 0°C for 10 minutes 261 mg 3-bromoethyl-5-(5-chloror-thiophen-2-yl)-isoxazole were added. The reaction mixture was allowed to warm up to room temperature and stirred for 2 hours. After removing of the solvent under reduced pressure the residue was purified by chromatography on silica gel eluting with a dichloromethane/ methanol gradient.
Yield: 80 mg MS(ES$^+$): m/e = 374, chloro pattern.
1H-NMR (400 MHz, DMSO/TMS): $\delta$ = 8.54 (d, 1H); 8.13 (d, 1H); 7.58 (d, 1H); 7.43 (s, 1H); 7.39 (dd, 1H); 7.26 (d, 1H); 7.73 (s, 1H); 5.98 (s, 2H); 3.90.(s, 3H).

(iv) 1-[5-(5-Chloro-thiophen-2-yl)-isoxazol-3-ylmethyl]-1H-pyrrolo[3,2-b]pyridine-2- carboxylic acid

A solution of 75 mg 1H-pyrrolo[3,2-b]pyridine-2-carboxylic acid methyl ester and 9.6 mg lithium hydroxide in a mixture of 3 ml tetrahydrofuran and 1ml water was stirred for 2 hours at room temperature. After acidifying with 6 N hydrochloric acid to pH 2 the solvent of the mixture was removed under reduced pressure. The resulting residue was purified by chromatography on silica gel eluting with a ethyl acetate/ methanol gradient with 0.1% water. Yield: 50 mg MS(ES$^+$): m/e=360, chloro pattern.
1 H-NMR (400 MHz, DMSO/TMS): $\delta$ = 8.45 (d, 1H); 7.84 (d, 1H); 7.53 (d, 1H); 7.22 (d, 1H); 7.15 (dd, 1H); 6.94 (s, 1H); 6.60 (s, 1H); 6.14 (s, 2H).

(v) 1-[5-(5-Chloro-thiophen-2-yl)-isoxazol-3-ylmethyl]-1H-pyrrolo[3,2-b]pyridine-2- carboxylic acid (1-isopropyl-piperidin-4-yl)-amide

To a suspension of 50 mg 1-[5-(5-chloro-thiophen-2-yl)-isoxazol-3-ylmethyl]-1H-pyrrolo[3,2-b]pyridine-2- carboxylic acid, 36 mg 1-isopropyl-piperidin-4-ylamine hydrochloride and 35 mg bis(2-oxo-3-oxazolidinyl)phosphinic chloride in 1 ml dichloromethane, 77 $\mu$l triethylamine were added at room temperature and the mixture was stirred for 16 hours. After removing of the solvent under reduced pressure, the residue was purified by preparative HPLC (C18 reverse phase column, elution with a water/ acetonitrile gradient with 0.1% trifluoroacetic acid). The fractions containing the product were evaporated and lyophilized to yield a white residue which was partitioned between 5 ml aqueous 0.1 N sodium hydroxide solution and 5 ml ethyl acetate. The organic layer was washed with additional water and then dried over sodium sulphate. After filtration and removal of the solvent under reduced pressure, a white solid was obtained.
Yield: 10 mg MS(ES$^+$): m/e = 484, chloro pattern.
1 H-NMR (500 MHz, DMSO/TMS): $\delta$ = 8.53 (d, 1H); 8.46 (d, 1 H); 8.03 (d, 1H); 7.57 (d, 1H); 7.32 (s, 1H); 7.28 (dd, 1H); 7.26 (d, 1H); 6.65 (s, 1H); 5.93 (s, 2H); 3.75 (m, 1H); 2.80 (m, 2H); 2.70 (m, 1 H); 2.17 (m, 2H); 1.80 (m, 2H); 1.53 (m, 2H); 0.96 (d. 6H)

Example 6: 1-[5-(5-Chloro-thiophen-2-yl)-isoxazol-3-ylmethyl]-2-(1-isopropyl-piperidin-4-ylcarbamoyl)-1H-pyrrolo[3,2-b]pyridine-5-carboxylic acid

(i) 5-Amino-6-bromo-pyridine-2-carboxylic acid methyl ester

To a solution of 5.00 g 5-amino-pyridine-2-carboxylic acid methyl ester in 75 ml of a 48% aqueous solution of hydrobromic acid, 3.39 ml of a 32% aqeous solution of hydrogen peroxide were added. The mixture was stirred at room temperature for 2 hours, then additional 0.80 ml hydrogen peroxide solution were added. After stirring for 1 hour the reaction mixture was cooled down and brought to pH 8 by addition of concentrated aqueous ammonia. The mixture was extracted with 300 ml ethyl acetate. The aqueous layer was washed with additional ethyl acetate and then the combined organic phases were dried over sodium sulfate. After filtration the solvent was removed under reduced pressure and the residue was purified by chromatography on silica gel eluting with a n-heptane/ ethyl acetate gradient. Yield: 2.83 g MS(ES$^+$): m/e= 231.
1 H-NMR (400 MHz, DMSO/TMS): $\delta$ = 7.80 (d, 1 H); 7.10 (d, 1H); 6.37 (s, 2H); 3.80 (s, 3H)

(ii) 1 H-Pyrrolo[3,2-b]pyridine-2,5-dicarboxylic acid 5-methyl ester

The following compound was prepared in analogy to example 5 by using 5-amino-6-bromo-pyridine-2-carboxylic acid methyl ester instead of 2-bromo-pyridin-3-ylamine. MS(ES$^+$): m/e= 221.
1H-NMR (400 MHz, DMSO/TMS): $\delta$ = 11.80 (s, 1H); 7.89 (d, 1H); 7.84 (d, 1H); 6.93 (s, 1H); 3.88 (s, 3H).

(iii) 2-(1-Isopropyl-piperidin-4-ylcarbamoyl)-1H-pyrrolo[3,2-b]pyridine-5-carboxylic acid methyl ester

To a solution of 140 mg 1H-pyrrolo[3,2-b]pyridine-2,5-dicarboxylic acid 5-methyl ester in 1.4 ml N,N-dimethylformamide and 0.35 ml triethylamine, 164 mg 1-isopropyl-piperidin-4-ylamine hydrochloride and 161 mg bis(2-oxo-3-oxazolidinyl)phophinic chloride were added at room temperature and the mixture was stirred for 1 hour. After removing of the solvent under reduced pressure the residue was purified by preparative HPLC (C18 reverse phase column, elution with a water/ acetonitrile gradient with 0.1% trifluoroacetic acid). The fractions containing the product were evaporated and lyophilized to yield a white solid. The product was obtained as its trifluoroacetate salt. Yield: 112 mg MS(ES$^+$): m/e = 345.

1H-NMR (400 MHz, DMSO/TMS): $\delta$ = 12.20 (s, 1 H); 9.10 (s, 1 H); 8.79 (d, 1 H); 7.93 (m, 2H); 7.42 (s, 1H); 4.14 (m, 1H); 3.90 (s, 3H); 3.47 (m, 3H); 3.15 (m, 2H); 2.15 (m, 2H); 1.88 (m, 2H); 1.28 (d. 6H).

(iv) 1-[5-(5-Chloro-thiophen-2-yl)-isoxazol-3-ylmethyl]-2-(1-isopropyl-piperidin-4-ylcarbamoyl)-1 H-pyrrolo[3,2-b]pyridine-5-carboxylic acid methyl ester

To a solution of 60 mg 2-(1-isopropyl-piperidin-4-ylcarbamoyl)-1H-pyrrolo[3,2-b]pyridine-5-carboxylic acid methyl ester in 2 ml N,N-dimethylformamide, 4 mg sodium hydride (95%) were added at 0°C. After stirring at 0°C for 10 minutes 53 mg 3-bromomethyl-5-(5-chloro-thiophen-2-yl)-isoxazole were added and the mixture was stirred for 2 hours at room temperature. After removing the solvent under reduced pressure the residue was purified by preparative HPLC (C18 reverse phase column, elution with a water/ acetonitrile gradient with 0.1% trifluoroacetic acid). The fractions containing the product were evaporated and lyophilized to yield a white solid. The product was obtained as its trifluoroacetate salt. Yield: 50 mg MS(ES$^+$): m/e= 542, chloro pattern.

1H-NMR (400 MHz, DMSO/TMS): $\delta$ = 8.93 (m, 2H); 8.25 (d, 1H); 8.04 (d, 1H); 7.55 (d, 1H); 7.42 (s, 1H); 7.28 (d, 1H); 6.69 (s, 1H); 5.97 (s, 2H); 4.07 (m, 1H); 3.91 (s, 3H); 3.45 (m, 2H); 3.10 (m, 2H); 2.10 (m, 3H); 1.83 (m, 2H); 1.25 (d. 6H).

(v) 1-[5-(5-Chloro-thiophen-2-yl)-isoxazol-3-ylmethyl]-2-(1-isopropyl-piperidin-4-ylcarbamoyl)-1 H-pyrrolo[3,2-b]pyridine-5-carboxylic acid

To a solution of 50 mg 1-[5-(5-chloro-thiophen-2-yl)-isoxazol-3-ylmethyl]-2-(1-isopropyl-piperidin-4- ylcarbamoyl)-1H-pyrrolo[3,2-b]pyridine-5-carboxylic acid methyl ester in 2 ml THF and 1 ml water 3.6 mg lithium hydroxide were added and the mixture was stirred at room temperature for 2 hours. Then the reaction mixture was acidified with 6 N hydrochloric acid to pH 3 and the solvent was removed under reduced pressure. The resulting residue was purified by preparative HPLC (C18 reverse phase column, elution with a water/ acetonitrile gradient with 0.1 % trifluoroacetic acid). The fractions containing the product were evaporated and lyophilized to yield a white solid. The product was obtained as its trifluoroacetate salt.

Yield: 25 mg MS(ES+): m/e= 528, chloro pattern.

1 H-NMR (500 MHz, DMSO/TMS): $\delta$ = 13.00 (s, 1 H); 8.90 (m, 2H); 8.23 (d, 1 H); 8.03 (d, 1 H); 7.56 (d, 1 H); 7.42 (s, 1 H); 7.28 (d, 1 H); 6.69 (s, 1 H); 5.98 (s, 2H); 4.07 (m, 1 H); 3.45 (m, 2H); 3.10 (m, 2H); 2.10 (m, 3H); 1.83 (m, 2H); 1.25 (d. 6H).

Pharmacological testing

The ability of the compounds of the invention to inhibit factor Xa or factor VIIa or other enzymes like thrombin, plasmin, or trypsin can be assessed by determining the concentration of the compound of the invention that inhibits enzyme activity by 50 %, i. e. the IC50 value, which was related to the inhibition constant Ki. Purified enzymes were used in chromogenic assays. The concentration of inhibitor that causes a 50 % decrease in the rate of substrate hydrolysis was determined by linear regression after plotting the relative rates of hydrolysis (compared to the uninhibited control) versus the log of the concentration of the compound of the invention For calculating the inhibition constant Ki, the IC50 value was corrected for competition with substrate using the formula

$$Ki = IC50 / \{1 + (substrate\ concentration\ /\ Km)\}$$

wherein Km is the Michaelis-Menten constant (Chen and Prusoff, Biochem. Pharmacol. 22 (1973), 3099-3108; I. H. Segal, Enzyme Kinetics, 1975, John Wiley & Sons, New York, 100-125; which were incorporated herein by reference).

### a) Factor Xa Assay

In the assay for determining the inhibition of factor Xa activity TBS-PEG buffer (50 mM Tris-HCl, pH 7.8, 200 mM NaCl, 0.05 % (w/v) PEG-8000, 0.02 % (w/v) NaN3) was used. The IC50 was determined by combining in appropriate wells of a Costar half-area microtiter plate 25 μl human factor Xa (Enzyme Research Laboratories, Inc.; South Bend, Indiana) in TBS-PEG; 40 μl 10 % (v/v) DMSO in TBS-PEG (uninhibited control) or various concentrations of the compound to be tested diluted in 10 % (v/v) DMSO in TBS-PEG; and substrate S-2765 (N(α)-benzyloxycarbonyl-D-Arg-Gly-L-Arg-p-nitroanilide; Kabi Pharmacia, Inc.; Franklin, Ohio) in TBS-PEG.

The assay was performed by pre-incubating the compound of the invention plus enzyme for 10 min. Then the assay was initiated by adding substrate to obtain a final volume of 100 μl. The initial velocity of chromogenic substrate hydrolysis was measured by the change in absorbance at 405 nm using a Bio-tek Instruments kinetic plate reader (Ceres UV900HDi) at 25 °C during the linear portion of the time course (usually 1.5 min after addition of substrate). The enzyme concentration was 0.5 nM and substrate concentration was 140 μM.

### b) Factor VIIa Assay

The inhibitory activity towards factor VIIa/tissue factor activity was determined using a chromogenic assay essentially as described previously (J. A. Ostrem et al., Biochemistry 37 (1998) 1053-1059 which was incorporated herein by reference). Kinetic assays were conducted at 25 °C in half-area microtiter plates (Costar Corp., Cambridge, Massachusetts) using a kinetic plate reader (Molecular Devices Spectramax 250). A typical assay consisted of 25 μl human factor VIIa and TF (5 nM and 10 nM, respective final concentration) combined with 40 μl of inhibitor dilutions in 10% DMSO/TBS-PEG buffer (50 mM Tris, 15 mM NaCl, 5 mM CaCl2, 0.05 % PEG 8000, pH 8.15). Following a 15 minutes preincubation period, the assay was initiated by the addition of 35 μl of the chromogenic substrate S-2288 (D-Ile-Pro-Arg-p-nitroanilide, Pharmacia Hepar Inc., 500 μM final concentration). The results (inhibition constants Ki (FXa) for inhibition of factor Xa) are shown in Table 1.

Table 1:

| Example | Ki(FXa) [μM] |
|---------|--------------|
| 1 | 0.006 |
| 2 | 0.055 |
| 3 | 0.067 |
| 4 | 0.070 |
| 5 | 0.004 |
| 6 | 0.010 |

## Claims

1. A compound in all its stereoisomeric forms and mixtures thereof in any ratio, and its physiologically tolerable salts, wherein the compound is

   1-[5-(5-Chloro-thiophen-2-yl)-isoxazol-3-ylmethyl]-1H-pyrrolo[2,3-b]pyridine-2-carboxylic acid (1-isopropyl-piperidin-4-yl)-amide,
   1-[5-(5-Chloro-thiophen-2-yl)-isoxazol-3-ylmethyl]-2-(1-isopropyl-piperidin-4-ylcarbamoyl)-1 H-pyrrolo[2,3-b]pyridine-5-carboxylic acid methyl ester,
   1-[5-(5-Chloro-thiophen-2-yl)-isoxazol-3-ylmethyl]-2-(1-isopropyl-piperidin-4-ylcarbamoyl)-1 H-pyrrolo[2,3-b]pyridine-5-carboxylic acid,
   1-[5-(5-Chloro-thiophen-2-yl)-isoxazol-3-ylmethyl]-1H-pyrrolo[2,3-b]pyridine-2,5-dicarboxylic acid 5-amide 2-[(1-isopropyl-piperidin-4-yl)-amide],
   1-[5-(5-Chloro-thiophen-2-yl)-isoxazol-3-ylmethyl]-1H-pyrrolo[3,2-b]pyridine-2-carboxylic acid (1-isopropyl-piperidin-4-yl)-amide, or
   1-[5-(5-Chloro-thiophen-2-yl)-isoxazol-3-ylmethyl]-2-(1-isopropyl-piperidin-4-ylcarbamoyl)-1H-pyrrolo[3,2-b]pyridine-5-carboxylic acid ,

**2.** A pharmaceutical preparation, comprising at least one compound as claimed in claim 1 in all its stereoisomeric forms and mixtures thereof in any ratio and/or its physiologically tolerable salts and a pharmaceutically acceptable carrier.

**Patentansprüche**

**1.** Verbindung in allen ihren stereoisomeren Formen und Mischungen davon in beliebigem Verhältnis und physiologisch verträgliche Salze davon, wobei sich bei der Verbindung um

1-[5-(5-Chlorthiophen-2-yl)isoxazol-3-ylmethyl]-1H-pyrrolo[2,3-b]pyridin-2-carbonsäure(1-isopropylpiperidin-4-yl)amid,
1-[5-(5-Chlorthiophen-2-yl)isoxazol-3-ylmethyl]-2-(1-isopropylpiperidin-4-ylcarbamoyl)-1H-pyrrolo-[2,3-b]pyridin-5-carbonsäuremethylester,
1-[5-(5-Chlorthiophen-2-yl)isoxazol-3-ylmethyl]-2-(1-isopropylpiperidin-4-ylcarbamoyl)-1H-pyrrolo-[2,3-b]pyridin-5-carbonsäure,
1-[5-(5-Chlorthiophen-2-yl)isoxazol-3-ylmethyl]-1H-pyrrolo[2,3-b]pyridin-2,5-dicarbonsäure-5-amid-2-[(1-isopropylpiperidin-4-yl)amid],
1-[5-(5-Chlorthiophen-2-yl)isoxazol-3-ylmethyl]-1H-pyrrolo[3,2-b]pyridin-2-carbonsäure(1-isopropylpiperidin-4-yl)amid oder
1-[5-(5-Chlorthiophen-2-yl)isoxazol-3-ylmethyl]-2-(1-isopropylpiperidin-4-ylcarbamoyl)-1H-pyrrolo-[3,2-b]pyridin-5-carbonsäure,

handelt.

**2.** Pharmazeutische Zubereitung, umfassend mindestens eine Verbindung nach Anspruch 1 in allen ihren stereoisomeren Formen und Mischungen davon in beliebigem Verhältnis und/oder physiologisch verträgliche Salze davon und einen pharmazeutisch unbedenklichen Träger.

**Revendications**

**1.** Composé, sous toutes ses formes stéréoisomères et des mélanges de celles-ci en toutes proportions et/ou ses sels physiologiquement tolérables **caractérisé en ce que** le composé est le (1-isopropyl-pipéridin-4-yl)-amide d'acide 1-[5-(5-chlorothiophén-2-yl)-isoxazol-3-ylméthyl]-1H-pyrrolo[2,3-b]pyridine-2-carboxylique, l'ester méthylique d'acide 1-[5-(5-chlorothiophén-2-yl)-isoxazol-3-ylméthyl]-2-(1-isopropylpipéridin-4-ylcarbamoyl)-1H-pyrrolo[2,3-b]pyridine-5-carboxylique, l'acide 1-[5-(5-chlorothiophén-2-yl)-isoxazol-3-ylméthyl]-2-(1-isopropylpipéridin-4-ylcarbamoyl)-1H-pyrrolo[2,3-b]pyridine-5-carboxylique, le 5-amide 2-[(1-isopropyl-pipéridin-4-yl)-amide d'acide 1-[5-(5-chlorothiophén-2-yl)-isoxazol-3-ylméthyl]-1H-pyrrolo[2,3-b]pyridine-2,5-dicarboxylique, le (1-isopropyl-pipéridin-4-yl)-amide d'acide 1-[5-(5-chlorothiophén-2-yl)-isoxazol-3-ylméthyl]-1H-pyrrolo[3,2-b]pyridine-2-carboxylique, ou l'acide 1-[5-(5-chlorothiophén-2-yl)-isoxazol-3-ylméthyl]-2-(1-isopropyl-pipéridin-4-ylcarbamoyl)-1H-pyrrolo[3,2-b]pyridine-5-carboxylique.

**2.** Préparation pharmaceutique, comprenant au moins un composé selon la revendication 1 sous toutes ses formes stéréoisomères et des mélanges de celles-ci en toutes proportions et/ou ses sels physiologiquement tolérables et un support pharmaceutiquement acceptable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 987274 A
- WO 9529189 A
- WO 9206711 A
- WO 9612800 A
- WO 9747651 A
- WO 0107436 A2

**Non-patent literature cited in the description**

- **A. E. P. ADANG ; J. B. M. REWINKEL.** *Drugs of the Future,* 2000, vol. 25, 369-383
- Indoles, Part One. 1972, vol. 25
- The Chemistry of Heterocyclic Compounds. John Wiley & Sons
- **R.E. WILLETTE.** *Advances in Heterocyclic Chemistry,* 1968, vol. 9, 27
- **J.-Y. MÉROUR.** *Curr. Org. Chem.,* 2001, vol. 5, 471
- **H. DÖPP et al.** Methoden der Organischen Chemie. Georg Thieme Verlag, 1994, vol. E6a,b
- **CHEN ; PRUSOFF.** *Biochem. Pharmacol.,* 1973, vol. 22, 3099-3108
- **I. H. SEGAL.** Enzyme Kinetics. John Wiley & Sons, 1975, 100-125
- **J. A. OSTREM et al.** *Biochemistry,* 1998, vol. 37, 1053-1059